(19) Europäisches Patentamt
European Patent Office
Office européen des brevets

(11) **EP 4 437 991 A1**

(12) **EUROPEAN PATENT APPLICATION**

(43) Date of publication:
**02.10.2024 Bulletin 2024/40**

(21) Application number: **23166058.0**

(22) Date of filing: **31.03.2023**

(51) International Patent Classification (IPC):
**A61B 34/10** (2016.01)    **A61B 18/14** (2006.01)
**A61B 34/20** (2016.01)    **A61B 90/00** (2016.01)

(52) Cooperative Patent Classification (CPC):
**A61B 34/10;** A61B 18/1492; A61B 34/25;
A61B 2018/00577; A61B 2018/00613;
A61B 2018/00898; A61B 2034/104;
A61B 2034/107; A61B 2034/2051;
A61B 2034/2065; A61B 2090/374; A61B 2090/376;
A61B 2090/3762; A61B 2090/3764; A61B 2090/378

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **CIANCARELLA, Martina**
  **Eindhoven (NL)**
• **ISOLA, Alfonso Agatino**
  **Eindhoven (NL)**
• **HAUTVAST, Guillaume Leopold Theodorus
  Frederik**
  **5656AG Eindhoven (NL)**

(74) Representative: **Philips Intellectual Property &
Standards**
**High Tech Campus 52**
**5656 AG Eindhoven (NL)**

(54) **PLANNING AND GUIDANCE FOR COMPOSITE ABLATIONS**

(57)    A system (12), comprising: a memory (20) comprising instructions (32); and one or more processors configured by the instructions to: commission plural ablation zones (82, 84) for a target region (58A), wherein at least one of the plural ablation zones comprises a composite ablation zone based on a blending of a group of simulated ablation zones using implicit functions.

FIG. 8

**EP 4 437 991 A1**

**Description**

FIELD OF THE INVENTION

The present invention is generally related to therapy treatment planning and guidance, and more particularly, to composite ablation therapy planning and guidance.

BACKGROUND OF THE INVENTION

In the field of interventional oncology, percutaneous ablation, including thermal and non-thermal based ablation, is an interventional cancer treatment option that has seen a significant increase in adoption in the past decade, and is predicted to continue to grow, at least in the near term, at a compound annual growth rate of approximately 8-10%. Thermal ablation can be delivered using various ablation modalities, including e.g., radiofrequency ablation (RFA), microwave ablation (MWA), high-intensity focused ultrasound (HIFU), focal laser ablation (FLA), and cryo-ablation. Non-thermal ablation techniques include irreversible electroporation (IRE), which uses non-thermal energy to create permanent nanopores in a cell membrane that disrupts cellular homeostasis and ultimately causes cell damage within an applied electric field.

In clinical practice, these ablation procedures consist of placing one or more ablation applicators inside or near a target region with the help of image guidance. Typically, physicians place these needle-like applicators while inspecting real-time ultrasound or interventional radiology images (CT/MR/CBCT), and determine the intended location for the resulting ablation based on information provided from the manufacturer, results in clinical trials, and personal experience. Current ablation planning offerings only support regular ellipsoidal ablation zones centered along an axis of a needle-like applicator, while the manufacturer data for a sub-set of the commercially available devices also specify simple (e.g., ellipsoidal) composite ablations, which include ablations achieved by simultaneously ablating with a plurality of applicators positioned in a fixed pattern with respect to each other. The planning of such composite ablations is a complex process, which typically requires applicator positioning in a fixed pattern in 3D space, whilst still enabling full tumor coverage, and sparing of surrounding critical structures. Thus, the clinical users (and automated methods alike) are confronted with a difficult planning problem with huge degrees of freedom and a quite large solution space cardinality. Subsequently, the clinical users face the challenge of placing the applicators accurately to achieve the ablation planned.

SUMMARY OF THE INVENTION

One object of the present invention is to improve upon existing systems in the planning and guidance of complex composite ablation zones in ablation therapy. To better address such concerns, in a first aspect of the invention, a system that commissions plural ablation zones for a target region is disclosed, wherein at least one of the plural ablation zones comprises a composite ablation zone based on a blending of a group of simulated ablation zones using implicit functions. By using a blending function, composite ablation zones derived from plural groups of applicators may be more accurately defined.

In one embodiment, the composite ablation zone comprises a non-ellipsoidal shape. Through use of the blending function, a more accurate representation of complex shapes such as found in MWA or IRE treatment techniques is realized when compared to binary union operators, the use of a blending function enabling more accurate ablation coverage and less risk to healthy tissue or anatomical structures, as well as applicability to planning of complex (e.g., non-ellipsoidal) ablation zones.

In one embodiment, a group of entry trajectories within a bounding box are shifted, rotated, or shifted and rotated to flexibly and more efficiently provide ablation coverage of a tumor corresponding to the target region.

In one embodiment, selection among a group of commissioned ablation zones is performed with an enforcement of requirements comprising establishing a threshold distance between all pairs of applicators corresponding to the plural commissioned ablation zones or reducing overlap between different groups of applicators to reduce treatment complexity.

These and other aspects of the invention will be apparent from and elucidated with reference to the embodiment(s) described hereinafter.

BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.

FIGS. 1A-1C are example representations of complex ablation zones that are represented by certain embodiments of an ablation therapy system, in accordance with an embodiment of the invention.

FIG. 2 is schematic diagram that illustrates an embodiment of an example ablation therapy system that facilitates planning and guidance to treat a tumor mass or lesion, in accordance with an embodiment of the invention.

FIG. 3 is a schematic diagram that illustrates a group of three applicators arranged in a triangle pattern with a pattern barycenter as the composite ablation entry trajectory origin, in accordance with an embodiment of the invention.

FIGS. 4A-4B are schematic diagrams of groups of entry trajectories based on repeated patterns of triangles, in accordance with an embodiment of the invention.

FIGS. 5A-5B are schematic diagrams of groups of three-applicator triangle patterns for free-hand ablation treatments relative to targets and at-risk organs, in accordance with an embodiment of the invention.

FIG. 6 is a schematic diagram that shows example entry trajectories based on x-direction and/or y-direction shifts for a two-applicator linear pattern in a grid usage scenario, in accordance with an embodiment of the invention.

FIGS. 7A-7C are schematic diagrams that show example composite ablation zones for two-applicator linear patterns alone and in combination with additional groups of applicators, in accordance with an embodiment of the invention.

FIG. 8 is a flow diagram that illustrates an embodiment of an example automated planning method, in accordance with an embodiment of the invention.

## DETAILED DESCRIPTION OF EMBODIMENTS

Disclosed herein are certain embodiments of an ablation therapy system and method that are used to plan and guide thermal or non-thermal ablation procedures, with the aim to make ablation procedures easier, more efficient, and more standardized, with the ultimate goal of contributing to better subject outcomes. The ablation therapy system includes automated and interactive planning methods, as well as device placement guidance that help a user perform an ablation procedure based on segmentations of the cancer lesion(s) and other anatomical structure segmentations. The ablation therapy system also selects from among a set of commissioned composite ablations for use during the ablation therapy treatment.

Digressing briefly, some existing ablation planning methods are typically based on manufacturer data on the expected ellipsoidal ablation zone achieved using an ablation device using plural combinations of power and duration. In the case of composite ablations, manufacturer data on the expected ellipsoidal composite ablation zones uses a combination of spacing between applicators, power, and duration. However, such composite ablation zones are rigidly based on a fixed pattern of the applicators, where each applicator is treated as an independent entity. Publications exist that describe or represent composite ablation zones using a simple binary union of ellipsoids, which do not accurately describe the expected ablation region provided on device documentations. Indeed, such techniques would not be usable for microwave (MW) and irreversible electroporation (IRE) devices, which deliver complex ablation zones with non-ellipsoidal shapes.

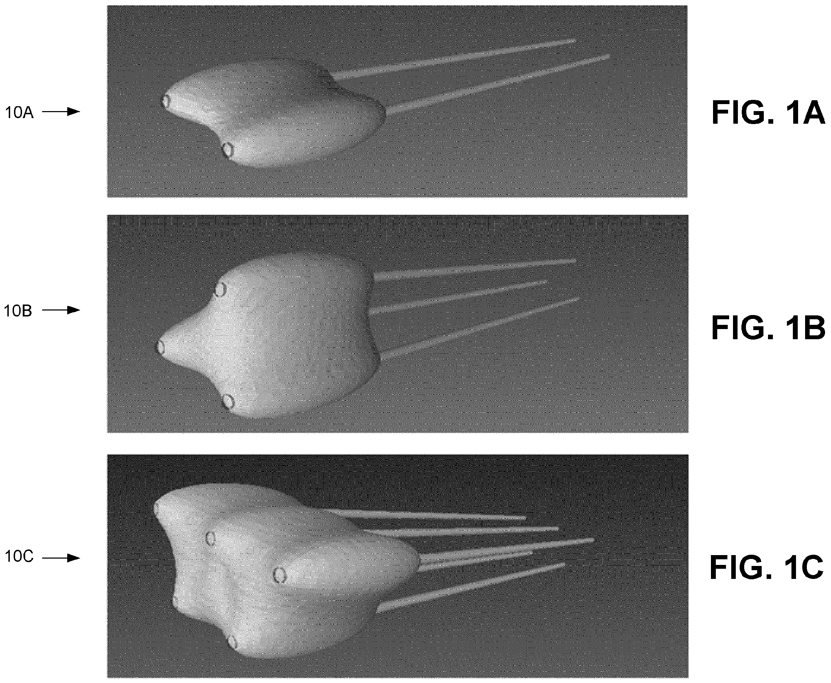

Explaining further, in the market, different probe models are given with datasheets indicating the expected geometry of both each single ablation zone delivered by a given probe configuration when used in isolation, and also the expected composite ablation zone delivered by different geometrical probe configurations. In some devices, the expected composite ablation zone has an ellipsoidal shape, however there are also other devices, for instance for MWA or IRE treatment, where the composite ablation zone can have quite complex shapes produced as a blending of the multiple ellipsoids delivered by each probe, as depicted by the example complex composite ablation zones 10A-10C in FIGS. 1A-1C, respectively. In contrast to some existing systems, certain embodiments of an ablation therapy system as disclosed herein blend implicit functions to represent composite ablation zones during a commissioning phase, which provides an advance in more accurately representing complex composite ablation zones such as those depicted in FIGS. 1A-1C delivered via multiple groups of probes.

Note that reference herein to a composite ablation zone refers to the expected total ablation zone shape delivered by a group or groups of executed applicators. Also, the term probes and applicators are used interchangeably herein.

Having summarized certain features of an ablation therapy system of the present disclosure, reference will now be made in detail to the description of an ablation therapy system as illustrated in the drawings. While an ablation therapy system will be described in connection with these drawings, there is no intent to limit it to the embodiment or embodiments disclosed herein. Further, although the description identifies or describes specifics of one or more embodiments, such specifics are not necessarily part of every embodiment, nor are all of any various stated advantages necessarily associated with a single embodiment. On the contrary, the intent is to cover alternatives, modifications and equivalents included within the principles and scope of the disclosure as defined by the appended claims. For instance, two or more embodiments may be interchanged or combined in any combination. Further, it should be appreciated in the context of the present disclosure that the claims are not necessarily limited to the particular embodiments set out in the description.

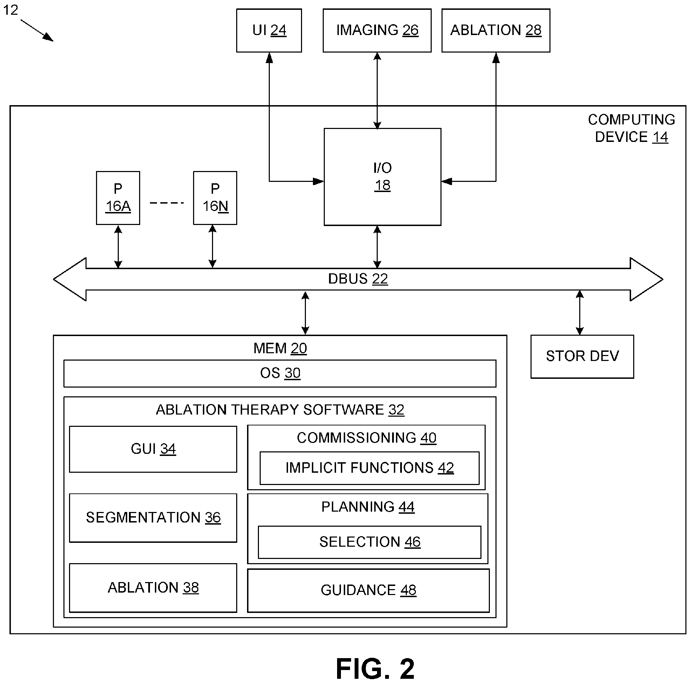

FIG. 2 illustrates an ablation therapy system 12 that facilitates the planning and guidance of one or more ablation protocols to guide treatment of a target region (e.g., a tumor mass or lesion, including any margin) in a subject. Note that reference herein is made to the ablation therapy system 12 responsible for treatment based on the planning and guidance functionality, though in some embodiments, the planning, guidance, and/or treatment may be implemented as separate systems in communication with each other. Successful treatment of large tumors can be achieved by planning ablation probe positions precisely through commissioning with the intent that no part of the tumor is left untreated, and accurately executing the plan. In general, the ablation therapy system 12 provides for automated and interactive planning of ablation therapy, guidance of applicator placement, and therapy ablation treatment based on the planning and guidance. The ablation plan ensures that a majority of (or potentially all) areas of the tumor are covered, and reports the number of ablations involved for an ablation treatment using a particular probe or group(s) of probes. The ablation therapy system 12 also utilizes selection techniques to minimize the number of ablations. Since the plan is quantitative, it can be carried out using a robot and/or by using registered image guidance, such as by quantitatively tracking the ablation probe.

In the depicted embodiment, functionality of the ablation therapy system 12 is implemented as a system of co-located software and hardware components collectively embodied as a computing device 14 (which may include a medical device) and plural sub-systems, wherein the computing device 14 is operatively connected to the plural sub-systems, as described below. In some embodiments, the computing device functionality may be embedded in one of the sub-systems, or one or more of the herein-described sub-system functionality may be integrated into fewer devices. It should be appreciated that, in some embodiments, functionality of the ablation therapy system 12 may be implemented via plural computing devices that are networked at a similar location or situated in different locations, potentially remote from each other, and connected via one or more networks. The computing device 14 includes one or more processors 16 (e.g., 16A...16N), input/output interface(s) 18, and memory 20, etc. coupled to one or more data busses, such as data bus 22. The processor(s) 16 may be embodied as a custom-made or commercially available processor, including a single or multi-core central processing unit (CPU), tensor processing unit (TPU), graphics processing unit (GPU), vector processing unit (VPU), or an auxiliary processor among several processors, a semiconductor-based microprocessor (in the form of a microchip), a macroprocessor, one or more application specific integrated circuits (ASICs), field programmable gate arrays (FPGUs), a plurality of suitably configured digital logic gates, and/or other existing electrical configurations comprising discrete elements both individually and in various combinations to coordinate the overall operation of the computing device 14.

The I/O interfaces 18 comprise hardware and/or software to provide one or more interfaces to various sub-systems, including to one or more user interface(s) 24, an imaging sub-system 26, and an ablation sub-system 28. The I/O interfaces 18 may also include additional functionality, including a communications interface for network-based communications. For instance, the I/O interfaces 18 may include a cable and/or cellular modem, and/or establish communications with other devices or systems via an Ethernet connection, hybrid/fiber coaxial (HFC), copper cabling (e.g., digital subscriber line (DSL), asymmetric DSL, etc.), using one or more of various communication protocols (e.g., TCP/IP, UDP, etc.). In general, the I/O interfaces 18, in cooperation with a communications module (not shown), comprises suitable hardware to enable communication of information via PSTN (Public Switched Telephone Networks), POTS, Integrated Services Digital Network (ISDN), Ethernet, Fiber, DSL/ADSL, Wi-Fi, cellular (e.g., 3G, 4G, 5G, Global System for Mobile Communications (GSM), General Packet Radio Service (GPRS),etc.), Bluetooth, near field communications (NFC), Zigbee, among others, using TCP/IP, UDP, HTTP, DSL.

The user interface(s) 24 may include a keyboard, scroll-wheel, mouse, microphone, immersive head set, display device(s), etc., which enable input and/or output by or to a user, and/or visualization to a user. In some embodiments, the user interface(s) 24 may cooperate with associated software to enable augmented reality or virtual reality. The user interface(s) 24, when comprising a display device, enables the display of segmented anatomical structure(s), including tumor(s), and the virtual (e.g., simulated) and actual placement of applicators and identification of ablation zones and ablation zone coverage, as well as virtual bounding boxes. In some embodiments, the user interface(s) 24 may be coupled directly to the data bus 22.

The imaging sub-system 26 includes one or more imaging sub-systems and/or image storage sub-systems that are used to enable visualization of tumors, virtual or actual applicator placement, and/or single or composite ablation zones during pre- and per-operative (e.g., during actual applicator placement and/or ablation treatment) imaging. The imaging sub-system 26 may include ultrasound imaging (e.g., 2D/3D in real-time), fluoroscopy (real-time), magnetic resonance (e.g., MR, in 2D/3D real-time or static), computed tomography (e.g., CT (3D static)), cone beam CT (e.g., CBCT (3D static)), single photon emission CT (SPECT), and/or positron emission tomography (PET). In some embodiments, the images may be retrieved from a picture archiving and communication systems (PACS) or any other suitable imaging component or delivery system.

The ablation sub-system 28 may include any one of various ablation modalities, including radiofrequency ablation (RFA), microwave ablation (MWA), high-intensity focused ultrasound (HIFU), focal laser ablation (FLA), irreversible electroporation (IRE), and cryo-ablation. In some embodiments, software in memory 20 may be used to interface with the ablation sub-system 28 to adjust settings (e.g., probe distance, power, duration), track positioning of the applicators, and/or in the case of robotic-based ablation therapy, control the actual placement and energizing of the applicators.

The memory 20 may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 20 may store a native operating system, one or more native applications, emulation systems, or emulated applications for any of a variety of operating systems and/or emulated hardware platforms, emulated operating systems, etc. In some embodiments, a separate storage device (STOR DEV) may be coupled to the data bus 22 or as a network-connected device (or devices) via the I/O interfaces 18 and one or more networks. The storage device may be embodied as persistent memory (e.g., optical, magnetic, and/or semiconductor memory and associated drives). In some embodiments, the storage device or memory 20 may store manufacturer data pertaining to various applicators and applicator patterns.

In the embodiment depicted in FIG. 2, the memory 20 comprises an operating system 30 (OS) (e.g., LINUX, macOS, Windows, etc.), and ablation therapy software 32. In one embodiment, the ablation therapy software 32 comprises a plurality of modules (e.g., executable code) co-hosted on the computing device 14, though in some embodiments, the modules may be distributed across various systems or sub-systems over one or more networks. The ablation therapy software 32 comprises a graphical user interface (GUI) module 34, a segmentation module 36, and an ablation module 38. The ablation therapy software 32 also comprises a commissioning module 40, which includes an implicit functions or algorithms module 42, and a planning module 44, which includes a selection module 46. The implicit functions module 42 comprises implicit functions or algorithms for blending of composite ablation zones. The ablation therapy software 32 further comprises a guidance module 48, which through cooperation with the user interface(s) 24, imaging sub-system 26, and ablation sub-system 28, among other modules, guides placement of the applicators via overlay graphics on top of images. Note that functionality of the modules of the ablation therapy software 32 may be shared amongst each other. In some embodiments, there may be fewer or additional modules. For instance, functionality of some modules may be combined, or additional functionality may be implemented yet not shown, such as a communication module, security module, etc.

Functionality of one or more of the various modules is briefly explained here, with further description below. The GUI module 34 provides for reconstruction and rendering on the user interface(s) 24 (e.g., a display device) of image data received from imaging sub-system 26. Objects such as a lesion, organs, critical and/or healthy anatomical structures (or generally, regions not intended for ablation), may be segmented automatically using existing algorithms or by hand with drawing tools along the axes via the segmentation module 36. The segmentation module 36 produces a description of the volumetric regions associated with the specific objects according to existing methods. The GUI module 34 may also provide visualizations of ablation zones, applicators, and applicator placement for application to one or more target regions, whether virtually placed (e.g., pre-operative) or actually placed (during per-operative implementations).

The ablation module 38, in conjunction with the GUI module 34, provides for the display of user-selectable options for applicators and/or applicator zones. For instance, the ablation module 38 may store manufacturer data on expected ellipsoidal ablation zones based on different settings (e.g., power, duration, probe separation distance, etc.) for the ablation sub-system 28, and/or pre-defined (e.g., from manufacturer data) applicator patterns (e.g., single probe, two probe, three probe, etc.). In some embodiments, the ablation module 38 may track positioning of the applicators of the ablation sub-system 28 using electromagnetic or other types of sensors on or near the applicators, and/or use image analysis for tracking. The ablation module 38 controls applicators during ablation procedures based on the settings, and in some embodiments, may be used in conjunction with robotic controls to control the placement and activation of the applicators.

The commissioning module 40, in cooperation with the GUI module 34, may enable user-configured placement of applicators and/or ablation zones and/or, optionally, selection of pre-defined placement patterns. The commissioning module 40 further comprises the implicit functions module 42, which is used to support non-ellipsoidal composite ablation zones based on blending of ellipsoidal zones from the applicators of a group, as explained further below. The commissioning module 40 may operate in a grid or grid-less applicator environment. The commissioning module 40 may provide for fixed positioning (as prescribed by manufacturer data obtained, in one embodiment, by the ablation module 38) or as configured by a user (e.g., clinical user) via the GUI module 34.

The planning module 44 provides for the automated and/or interactive planning of single and composite ablations. For instance, based on segmented anatomical regions, the planning module 44 utilizes the commissioned data to precompute a plurality of (e.g., in some embodiments, all possible) probe configurations from which a preferred solution or plan is selected. The planning module 44 further comprises a selection module 46, which analyzes information associated with the tumor(s) and applicator positioning and ablation zones, and defines a set of ablation positions with orientations. In one embodiment, the selection module 46 comprises existing optimization functionality, which identifies the fewest (or fewer) number of ablations possible that cover the tumor region (e.g., tumor or tumor plus margin). In some embodiments, the selection module 46 identifies the ablation positions with orientations that spare the healthy tissue (e.g., that minimizes or reduces the risk of collateral damage). In some embodiments, additional object volumes are segmented that denote critical regions of tissue or bone that are not to be ablated, and the selection module 46 attempts to generate either the fewest ablations or minimize collateral damage, while also avoiding or reducing the risk of ablation of these regions. In some cases however, the selection module 46 produces unablated areas, whereupon the user is alerted and the regions can be displayed on the user interface(s) 24.

The guidance module 48 provides for user interactive guidance of applicator placement, as described below.

A general description of example operations of the ablation therapy software 32 is described below. After tumor and risk organ segmentation via segmentation module 36, the commissioning module 40 is used to enable commissioning under the direction or instruction of a user (e.g., clinical expert) via GUI module 34. For instance, during commissioning, the clinical expert decides which probes and probe settings to use. Probe setting may include probe model, geometry entry pattern (e.g., single probe, two probes in a linear pattern, three probes in a linear or triangular pattern, four probes in a square pattern, etc.), and expected ablation zone (e.g., ellipsoid or complex blended shape). In one embodiment, the ablation therapy software 32 may provide a commissioning GUI (e.g., via GUI module 34) for interaction with a clinical expert for complex blended shapes, where blending parameters may be manually tested, or in some instances, these blending parameters may be automatically computed if the user loads meshes or binary volumes representing an expected composite ablation zone per manufacturer data. Once commissioning is completed, operation by the ablation therapy software 32 resumes with the planning module 44. In one embodiment, the selection module 46 uses the commissioned data to precompute possible probe configurations from which a preferred solution or plan for treating a target region is selected. For instance, the selection module 46 may determine which of the blended ablation zones are to be selected or removed. As a simple illustration, and in the case of the selection module 46 comprising optimization functionality, an iterative solver of the selection module 46 may select a plan out of a plurality of (and in some embodiments, all possible) precomputed probe configurations (e.g., including single and/or multi-probe groups delivering a simple ellipsoid or blended ablation zones, respectively), such as a single probe delivering an ellipsoid ablation in one area of the target region and a triangular group of probes delivering a composite blended ablation at another area of the target region. In the case in which a grid template is used, the planning module 44 automatically computes a plurality of (and in some embodiments, all possible) insertion patterns and positions fitting or matching the grid openings/dimensions of the template, which would be an impractical endeavor for a user without the benefit of the ablation therapy software 32. Following the planning stage, operations resume with the guidance module 48 for guided physical placement of the probes, and then actual execution of ablation therapy.

Note that the memory 20 and storage device may each be referred to herein as a non-transitory, computer readable storage medium or the like.

Execution of the ablation therapy software 32 may be implemented by the one or more processors 16 under the management and/or control of the operating system 30.

When certain embodiments of the computing device 14 are implemented at least in part with software (including firmware), it should be noted that the ablation therapy software 32 can be stored on a variety of non-transitory computer-readable (storage) medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the computing device 14 are implemented at least in part with hardware, such functionality may be implemented with any or a combination of the following technologies, which are all already existing in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), TPUs, GPUs, and/or other accelerators/co-processors, etc.

One having ordinary skill in the art should appreciate in the context of the present disclosure that the example computing device 14 is merely illustrative of one embodiment, and that some embodiments of computing devices may comprise fewer or additional components, and/or some of the functionality associated with the various components depicted in FIG. 2 may be combined, or further distributed among additional modules or computing devices, in some embodiments. It should be appreciated that certain well-known components of computer systems are omitted here to avoid obfuscating more relevant features of the computing device 14.

Before commencing further particulars of certain embodiments of the ablation therapy system 12 and the ability of the system to accurately represent complex composite ablation zones, a brief description follows about certain requirements to fulfill when delivering a composite ablation zone as described on vendor datasheets. The definition or representation of composite ablation zones, also referred to as commissioning, prescribes the position and orientation of the applicators (or equivalently, probes) relative to the ablation zone, as well as the dimensions of the ablation zone. This commissioning may be performed as fixed (e.g., performed according to the manufacturer of the system) or configurable (e.g., performed by end-users of the system).

When configurable by the end-user, the system is equipped with a graphical user interface (GUI) (e.g., via GUI module 34, FIG. 2) that enables specifying the commissioning information, including the relative positioning of applicators and the composite ablation zone (e.g., tip offsets, spacing, etc.) and/or ablation zone dimensions (e.g., major and minor axes of an ellipsoid). The relative positioning of applicators and ablation zones can be expressed with respect to the center of the ablation zone or the tip location of any of the associated applicators. Further, the GUI is used to enable a user to virtually position a single probe or a group of probes that have been chosen during the commissioning phase, with feedback of the resulting ablation zones (e.g., composite or otherwise) presented on-the-fly to the user.

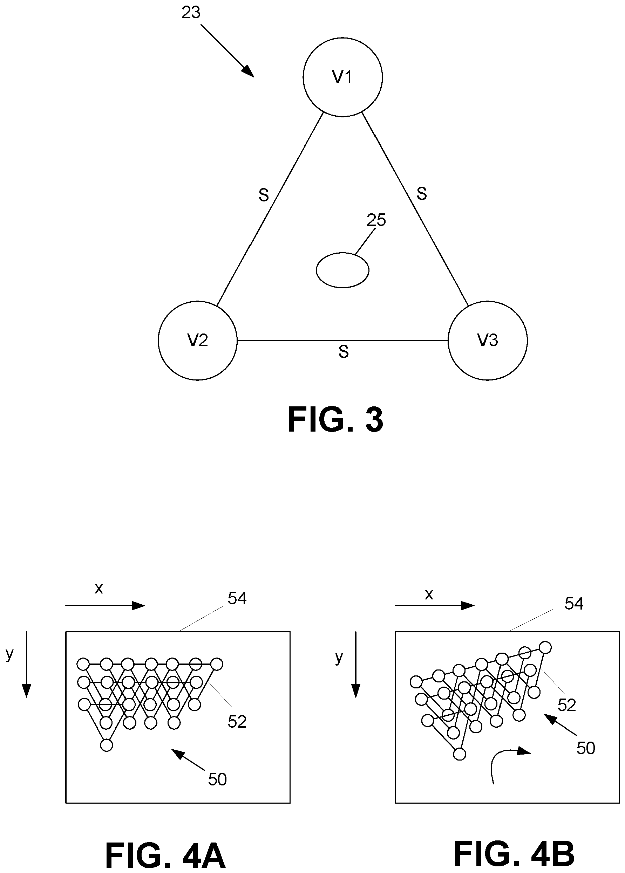

The user may be supported to specify composite ablation zones by a GUI (e.g., via GUI module 34 in conjunction with user interface(s) 24 as in FIG. 2) providing a CAD-like drawing environment enabling the relative positioning of applicators and composite ablation zone or a selection of pre-defined placement patterns, for which a limited number of parameters enables the relative positioning of applicators and composite ablation zone. The pre-defined placement patterns replicate information provided by the ablation device manufacturer, which includes, but is not limited to a line with applicators separated by distance, s (2 applicators), a triangle with sides of length s (3 applicators), a square with sides of length s (4 applicators), a trapezoid specified by a length and angle (4 applicators), etc. When the user selects one of these patterns, and provides values for the associated parameter(s), the system computes the relative positioning of the applicators with respect to the center of the ablation zone. For instance, and referring to FIG. 3, shown is a group of three applicators in the form of a triangle pattern 23. The three vertices V1, V2, and V3 represent respective applicator entry points (corresponding to entry trajectories), and the triangle pattern comprises a composite ablation zone entry trajectory 25 corresponding to the triangle barycentre. Parameter s indicates the length of each triangle side. As an example, for an insertion pattern with an equilateral triangle shape of side s, the ablation entry trajectory origin may be positioned at (x,y) = (0,0) and the origins of the three applicator entry trajectories (i.e., triangle vertices V1, V2 and V3) may be obtained as follows:

$$V1(x,y) = \left( -\frac{s}{2}, -\frac{1}{3}\sqrt{S^2 - (\frac{S}{2})^2} \right) \qquad \text{EQN. 1}$$

$$V2(x,y) = \left( \frac{s}{2}, -\frac{1}{3}\sqrt{S^2 - (\frac{S}{2})^2} \right) \qquad \text{EQN. 2}$$

$$V3(x,y) = \left( 0, \frac{2}{3}\sqrt{S^2 - (\frac{S}{2})^2} \right) \qquad \text{EQN. 3}$$

As described above, certain embodiments of an ablation therapy system (e.g., ablation therapy system 12, FIG. 2) support not only ellipsoidal but also non-ellipsoidal composite ablation zones, such as those achieved by IRE, by providing a commissioning GUI (e.g., a GUI rendered on a display device, such as via GUI module 34 and user interface(s) 24 in conjunction with commissioning module 40, as in FIG. 2) that enables describing a composite ablation zone as a combination of ellipsoids along with an additional blending parameter (e.g., via implicit functions module 42, as in FIG. 2). The blending of individual ellipsoids into a composite ablation zone may be based on the mathematical concept of implicit functions, as provided via algorithms in implicit functions module 42. An implicit function F(P) representing a surface specifies a position P to be on the surface if F(P) = 0. If the position is inside or outside the surface, F(P) < 0 or F(P) > 0, respectively. For an ellipsoidal surface around centroid Pc = (xc, yc, zc), the implicit function is defined by:

$$F(P) = F(x,y,z) = \frac{x-x_c}{r_x^2} + \frac{y-y_c}{r_y^2} + \frac{z-z_c}{r_z^2} - 1 \qquad \text{EQN. 4}$$

To calculate the composite ablation, the implicit functions of Ns individual ablation zones may be combined via another implicit function:

$$H(P) = 1 - \sum_{i=0}^{N_s} g(F_i(P)) = 0 \qquad \text{EQN: 5}$$

Here, the blending function g(x) can be defined in various ways, including, but not limited to:

$$g(x) = e^{-bx} \qquad\qquad \text{EQN: 6}$$

$$g(x) = \frac{a}{R^{n+1}}(x - R)^n(\frac{R}{a} - x)), \qquad\qquad \text{EQN: 7}$$

where b, a, R, and n are blending parameters, and where these two blending functions are used to enforce a smooth transition between two basic surfaces (e.g., two ellipsoids). The exponential blending function of equation 6 comprises parameter b, where b > 0 is a parameter to control the blending curve steepness (e.g., where b = 1 comprises two separated ellipses, b = 0.5 comprises slight blending, somewhat similar to an hourglass shape, and b = 0.3 comprises more extensive blending where the overall shape is more oblong with a slightly narrower middle region). Equation 7 comprises a distance-based blending function, where the function is true if x ≤ R, otherwise, g(x) = 0. In equation 7, a ∈ [0,1], n ∈ N*, and R > 0 is a blending radius. Here, the blending action vanishes when x > R, and parameter n adjusts the blending function by steps and parameter, a, realizes a fine-tuning in between the steps. The value(s) for the blending parameter(s) may be set directly by a value provided by the user, or indirectly by automatic adjustment of the blending parameter to mimic a composite ablation shape prescribed by a mesh provided by the user. For instance, the user may change one or more of the blending parameter values, and the resulting blended shape may be computed on-the-fly and the commissioning module 40 may, in cooperation with the GUI module 34, provide visual feedback of the newly blended shape. In some embodiments, the computed shape may be plotted next to the shape proposed by the manufacturer. In some embodiments, quality metrics may be provided (e.g., Dice coefficient) to show how the current blended shape overlaps with a reference shape. As to the mesh-based value determination approach, in instances where an ablation device manufacturer computes an expected composite ablation shape, such as via the use of bio-heat transfer modeling the probes and the anatomy, these computed composite ablation regions may be provided as meshes or binary masks. In such instances, blending function parameters may be inferred using, say, an I-squared optimization method or other methods that minimize or reduce the error between the computed meshes and a reference ground truth mesh provided by the manufacturer. Certain embodiments of an ablation therapy system 12 include an automatic planning component (e.g., via planning module 44, FIG. 2) to support a user (e.g., clinical expert) in establishing an ablation plan. The ablation planning selection problem generally consists of a preferred (e.g., depending on one or more user criteria), or in some embodiments, optimal selection of a set of applicators delivering single/regular and/or composite ablation zones to ablate the target region (e.g., tumor plus an optional margin) as well as possible (e.g., at 100% of its volume) while, at the same time, sparing nearby organs at risk and normal tissue. The automatic planning features of the planning module 44 may be implemented to support planning of composite ablations, as well as the planning of regular ablation zones achieved using a single applicator, for grid-based and free-hand ablation procedures. When a composite ablation zone is commissioned and used for a given treatment, multiple applicators are to be inserted in the subject's body according the defined placement pattern, and activated simultaneously to achieve the composite ablation zone within the target region. The delivered composite ablation zone resides along an entry trajectory that lies in-between the entry trajectories used for the insertion of applicators. Usually, this so-called composite ablation entry trajectory traverses through the center of the composite ablation zone, which coincides with the barycentre of the corresponding applicators. This composite ablation entry trajectory is oriented parallel to the direction of the corresponding applicators.

As described above, existing ablation planning generally treats each applicator as an independent entity for purposes of representing the ablation zone, or relies on binary unions of ellipsoids for composite ablation representation, which do not accurately describe the expected ablation region provided on device documentations, particularly in the case of complex composite ablation zones. In contrast, certain embodiments of an ablation therapy system 12 comprise an automatic planning component of the planning module 44 that supports composite ablation zones, and in particular, uses a group of entry trajectories that are computed for the composite ablation zones during commissioning, including the insertion paths used for positioning both the medical applicators and the corresponding composite ablation zone. More specifically, the ablation therapy system 12 disclosed herein defines (and improves over) pre-determined groups of applicators with different placement patterns, which are executable via possible grid holes (or for grid-less, via skin entry points, on virtual planes orthogonal to user provided directions).

With regard to operations involving an embodiment of the commissioning module 40, and referring to FIGS. 4A-4B, shown are a collective of plural groups 50 of entry trajectories based on, in this example, repeated patterns of triangles. Note that in some embodiments, other and/or additional patterns may be used, including a mix comprising single and multi-probe ablations. In particular, group 52 in this example comprises three applicators formed in an equilateral triangular pattern, with a plurality of such groups 50 arranged within a bounding box 54. Though bounding boxes 54 are shown partially filled with the collective groups 50, it should be appreciated that the depictions in FIGS. 4A-4B are merely showing a representative sample of triangle patterns, and that in practice, the bounding boxes 54 are occupied in the directions up to the respective border of the bounding box 54 based on a predefined or user-configured sampling technique (e.g., coarse sampling with fewer triangles, or fine sampling with a greater number of triangles and hence more aggressive overlap among the triangles). As similarly described in association with FIG. 3, the vertices of groups 52 are the entry points used by the applicators. The ablation zone entry origin lies along the barycenter of this pattern. The vertices of these groups 52 are considered collectively as a unique entity, delivered via a given geometrical placement pattern, and the group 52 delivers a blended composite ablation zone, where the ellipsoid zones of the vertices are blended to form the composite ablation zone shape. FIG. 4A shows the possibility of shifts in the x-direction (e.g., x = d/2) and/or y-direction (e.g., y = d/2), whereas FIG. 4B shows the shifts in the x and/or y direction and/or a roto-translation of the groups 50 and 52 as a collective entity (e.g., shifts or rotations of the groups 52 are shown, as well as the collective shifts/rotations of the groups 50 within the bounding box 54). Note that in this example of a triangle pattern, dimension d is the length of the equilateral triangle side (e.g., distance between probes), though the shift spacing may be provided via user input. Given a set of user insertion directions, groups 50 or 52 of entry trajectories may be shifted (e.g., in the x and/or y direction) within bounding boxes 54 encompassing 2D target projections computed over virtual planes perpendicular to the user directions, as shown in FIG. 4A. That is, FIG. 4A illustrates how the total set of triangular placement patterns covering a virtual box via shifts along the x-y directions may be computed. Moreover, as shown in FIG. 4B, groups 50 or 52 of insertion trajectories may be rigidly roto-translated within the set target bounding box 54. For instance, FIG. 4B illustrates how the total set 50 of placement patterns may be extended via roto-translation.

Accordingly, a large set of sampled group positions may be achieved setting an x and/or y shift and/or a rotation angle. Viewed according to the overall functioning of the ablation therapy software 32, various possible entry points for triangular groups of probes are commissioned. Based on manufacturer data and the commissioned single and/or composite ablation zones selected by the user for treatment (e.g., using different device settings to deliver simple and/or complex ablation zones, such as via linear patterns, triangle patterns, rectangle patterns, etc.), entry patterns of the groups of probes and the relative blending ablation zone for the groups is precomputed. This process may involve the translation/rotations described in association with FIGS. 4A-4B (e.g., the ablation zone shapes delivered by triangle patterns 50 within the bounding box 54 are identical in this example, allowing translation and/or rotation in space). Given the precomputed set of the possible blended ablation zones delivered by the groups of probes entered via multiple sampled entry points, and with tips positioned at multiple and different depths, processing resumes via the planning module 44 where selection of a set of probes among the commissioned probes is achieved. In one embodiment, the selection module 46 comprises a set-covering, greedy, iterative optimizer that selects a preferred set of probe groups (e.g., triangle patterns in this instance, though the preferred set in some scenarios may include different shapes, including triangles, squares, dots, etc.) to fully cover the tumor (target region) while sparing the risk organs nearby. In effect, the final treatment plan is the combination of the multiple probes' patterns of grouped applicators delivered over a single or multiple bounding boxes orthogonal to a single or multiple directions provided by the user. Note that selection of the preferred set may be an optimized or best set in some embodiments, or a set that is less than what may be considered as the best set yet suitably meets certain criteria. For instance, example criteria may include quantity or computational time of iterations in selection, anticipated time of treatment, the quantity, size, and/or maturity of the tumors, assessed or predicted risk of malignancy, confidence in such an assessment or prediction, subject and/or subject family history, among other factors established in the medical community and/or by the clinical expert. One or more of these factors may be considered and/or weighted relative to one or more other factors in a manner that might result in an outcome that is less than the best outcome as achieved by an optimizing function, yet preferred based on the established criteria. In some embodiments, a preferred solution may comprise greater subjectivity in considering an outcome than an optimization function, and/or in some embodiments, an optimizing function may be used yet constrained based on computational resources and/or computation time.

Note that in some embodiments, multiple bounding boxes that are orthogonal to the user-given entry directions may be used, and the shifts (translational or rotational) may be performed independently (e.g., an x-direction shift alone or in combination with a y-direction shift and/or rotational shift, a y-directional shift alone or in a combination with one or more other shifts, etc.). Further, it is noted that the bounding box 54 is a generalized concept for both grid- and grid-less scenarios. In the grid case, the bounding box 54 is given by the grid template geometry, and in the grid-less case, the bounding box 54 is virtually computed over a plane orthogonal to the user given directions.

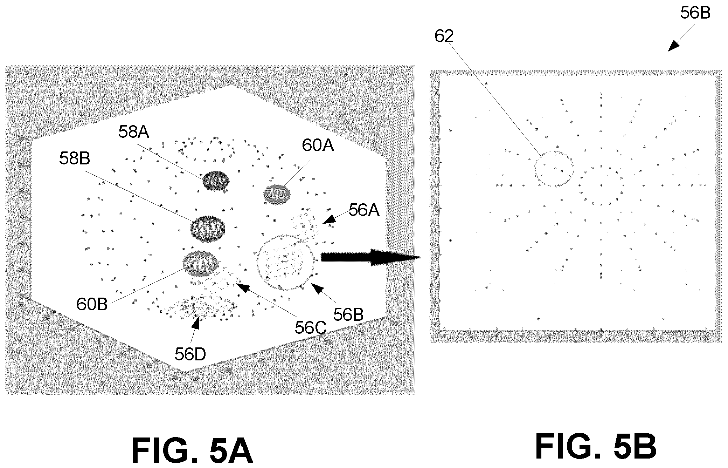

FIGS. 5A-5B further illustrate groups of three-applicator triangle patterns for free-hand ablation treatments relative to targets and at-risk organs. Referring to FIG. 5A, shown are different collective groups 56 (e.g., 56A-56D) with blended groups of a three applicator triangle pattern for free-hand ablation treatments. As similarly noted above, a different pattern or a mix of patterns may be used within one or more of the groups 56. In this example, meshes for two spherical targets 58A, 58B and two at-risk organs 60A, 60B are shown. In this case, two user-given directions are given to reach and ultimately treat the two target regions. That is, two user directions are provided as 3D unit vectors (not shown, but inferred from FIGS. 5A-5B), where for the user directions, two bounding boxes are computed, respectively encompassing the two targets 58A, 58B (and thus, four (4) groups 56A-56D). The groups of triangles 56A, 56B belong to the two bounding boxes lying on a plane orthogonal to the first of the user directions. A number of triangular groups of entry points among the groups 56 within target bounding boxes are sampled. In the close-up shown in FIG. 5B, a triangle pattern is encircled 62. That is, FIG. 5B illustrates a view that is orthogonal to the group 56B, and one of the triangles of the group 56B is encircled 62. Here, the three vertices (i.e. the three applicators' entry points) and the composite ablation zone's entry trajectory origin (i.e. triangle barycentre) are given. Stated otherwise, four of the dots encircled by 62 represent the three triangle vertices (e.g., the skin insertion points of the probes), and the barycentre represents the virtual entry point along which the composite blended ablation zone is to be positioned inside the body. More precisely, the simple (and/or complex) ablation zone is positioned along a virtual entry trajectory with the origin at the virtual skin entry point (e.g., triangle barycentre) and with the entry direction parallel to the probe(s) entry trajectory or trajectories. In case of a simple, single probe delivering a single ellipsoid, the ablation zone skin entry point and entry direction is identical or nearly to the probe entry point and entry direction. That is, for standard ellipsoids, the virtual ablation entry trajectory corresponds to the real entry trajectory of the probe.

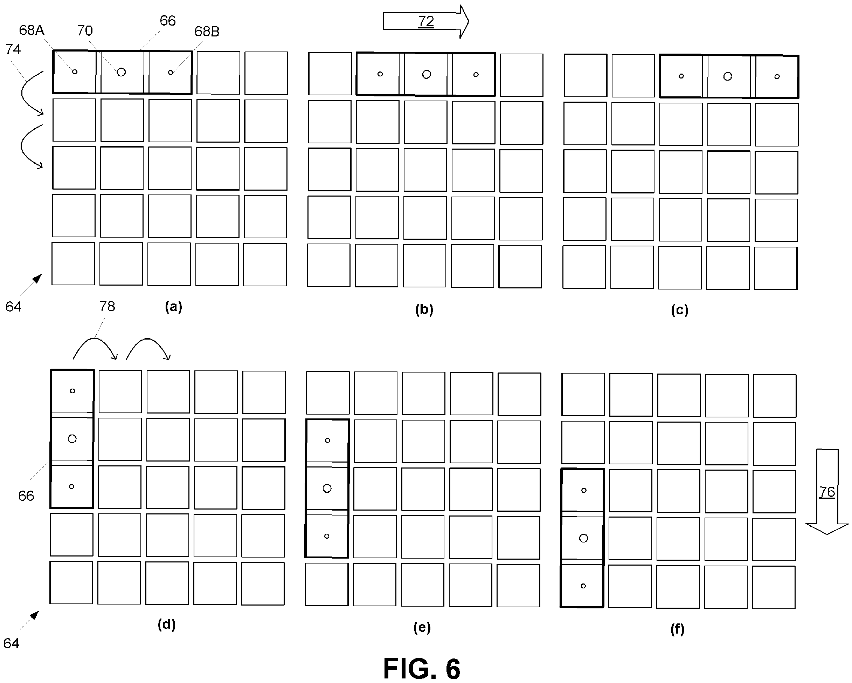

In a grid scenario, the sampling of groups of entry trajectories is strongly constrained by the regular grid geometry and the grid holes sampling value. For instance, an equilateral triangular pattern cannot be delivered via the grid templates currently supported by some systems, since an equilateral triangle has 60-degree angles, while a square grid has hole-hole diagonals at 45-degree angles. Differently, groups of 2-, 3- or 4-applicators with a linear/segment pattern may be delivered via a grid template if the relative distance of the applicators is a multiple of the grid holes spacing. FIG. 6 shows a simple and straight-forward way to determine placement pattern positions matching the grid holes of a grid or grid template, where the linear patterns match the grid hole spacing dimensions. For instance, and referring to FIG. 6(a-f), shown are example entry trajectories based on x-direction and/or y-direction shifts for a two-applicator linear pattern in a grid usage scenario. Focusing on FIG. 6(a) for the explanation of features for this diagram, with similar applicability to the other grid depictions in FIG. 6(b-f), shown is a grid or grid template 64 for which a pair of applicators or an applicator set (group) having a linear applicator pattern 66 is sampled. The applicator sets 66 in this example comprise the applicator trajectories 68A, 68B and the corresponding ablation trajectory 70. The applicator set 66 is sampled at a given x-y location of the grid 64 for a given row to derive an ablation configuration zone, then iteratively shifted, for instance, in the x-direction (as denoted by shift symbol 72). The shift 72 may be, in some instances 0.5 centimeters (cm), or whatever grid spacing is used, or a multiple of the grid hole spacing. The applicator set 66 is further sampled at this advanced position (FIG. 6(b)), and the process is repeated at the corresponding advanced positions for that row as shown in FIG. 6(c). This process may be repeated for one or more subsequent rows, as denoted by row-advance symbol 74. Referring to the grid templates 64 in FIG. 6(d-f), the applicator set 66 is sampled and shifted, as denoted by shift symbol 76, which again may be the grid hole spacing or multiples thereof) in the orthogonal direction (e.g., here, in the y-direction) in a similar iterative manner, and likewise, the sampling process continues to be performed for the columns as denoted by column-advance symbol 78. Note that shifts 72 and 76 may be performed according to a difference sequence (e.g., reversed, alternated, etc.) and/or rows and/or columns may be skipped, in some embodiments. In contrast to existing approaches, the ablation therapy software 32 (FIG. 2) automatically computes the groups of probes that match the grid spacing, and takes into account the blended composite ablation zones during selection. Existing techniques only consider each probe in isolation, and do not propose a mechanism to precompute the possible geometrical groups of probes matching the grid geometry (and do not consider blended shapes during selection). As explained above, the total composite ablation zone of such existing systems is described with a simple binary union operation over the ellipsoids, though this binary union is generally not a good approximation of the true composite ablated region.

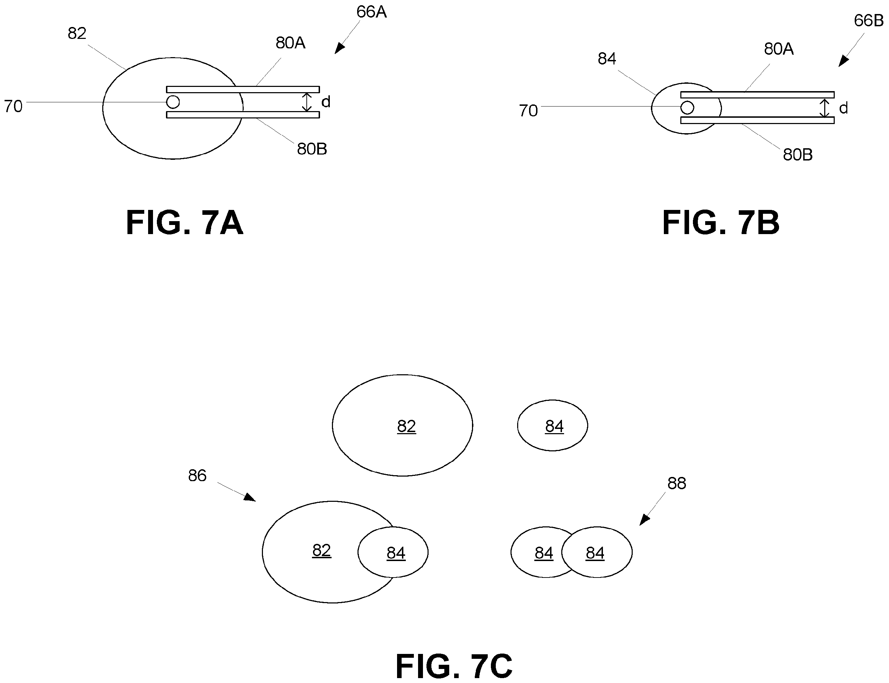

FIGS. 7A-7B show ablation shapes for a two-applicator linear (placement) pattern, such as those depicted in FIG. 6 (though not limited to grid scenarios), which includes a type of information that may be referenced in a device datasheet and stored by the ablation module 38 (FIG. 2). In this particular example, and referring to FIG. 7A, shown is an applicator set 66A comprising applicators 80A, 80B that may correspond to applicator trajectories 68A, 68B, respectively, as described in association with FIG. 6, and further shown is the ablation trajectory 70 for this particular set 66A. As explained above, though these features of the applicator set or group 66A are similar to those depicted in the grid-scenario of FIG. 6, it should be appreciated that the description above and below associated with FIGS. 7A-7C is applicable to grid and grid-less scenarios. Applicator separation dimension, d, may be applicable to the given applicator set, and in one example, may be 1.0 cm. FIG. 7B shows an applicator set or group 66B comprising applicators 80A, 80B that may correspond to applicator trajectories 68A, 68B as described in association with FIG. 6, and further shown is the ablation trajectory 70 for this particular set 66B. Notably, the patterns in FIGS. 7A and 7B deliver two different composite ablation zones 82 and 84, respectively, for two different device settings (e.g., power and time ON or duration at the given probe separation dimension). For instance, ablation shape 82 may be based on a higher power level provided via applicator set 66A when compared to the ablation shape 84 generated by applicator set 66B, with dimension d having a value of, for instance, 1.0 cm. In other words, applicator sets 66A and 66B may be similarly dimensioned though used at a different power and/or duration level to provide a different composite ablation shape/size. FIG. 7C shows the resulting composite ablation zones based on differences in settings and also example combinations of composite ablation zones 86 and 88 as selected by the selection module 46 (FIG. 2) to plan ablation therapy treatment. That is, aside from the individual composite ablation zones 82 and 84, additional groups of applicators using the same and/or different placement patterns may be combined by the selection module 46 to provide for further coverage, such as shown with the combined composite ablation zones 82 and 84. Though depicted in FIGS. 7A-7C using a combination of simple composite ablation zones 82 and 84 (e.g., shaped respectively as ellipses), the choice of ellipses as a shape is merely for illustration, and as should be appreciated, the group composite ablation zones may be ellipsoidal or non-ellipsoidal (e.g., blended shapes), such as shown in FIGS. 1A-1C. In some embodiments, the selection module 46 may take into account the geometrical information about the probe group(s) and the composite ablation zone when selecting a preferred plan (based on selection among the commissioned probes) covering a tumoral region.

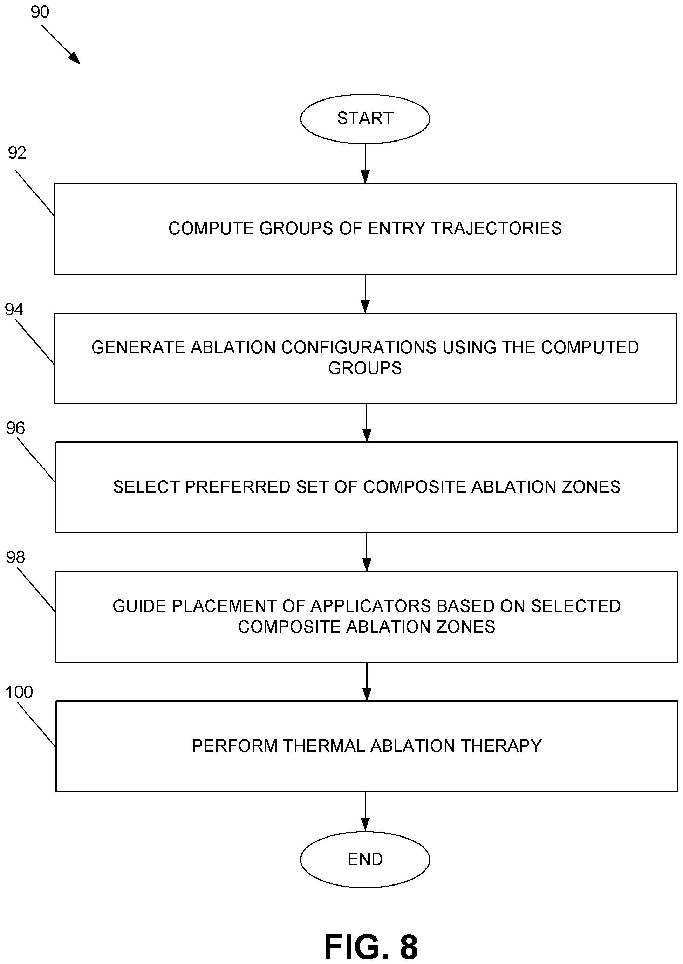

In view of the description above, it should be appreciated that certain embodiments of an ablation therapy system (e.g., ablation therapy system 12, FIG. 2) provide for ablation configuration generation, as described above, and automatic planning selection. FIG. 8 shows an embodiment of an example automated planning method 90. As described above, the method 90 computes groups of entry trajectories (92). The groups may be of the same pattern, or a mix of patterns. Shifts in the x and/or y direction, and/or roto-translational shifts may be used, as explained above in association with FIGS. 4A-4B. For grid applications, shifts according to grid hole spacing or multiples thereof in the x and/or y direction may be used. The method 90 further comprises generating ablation configurations using the computed groups (94). The final set of sampled groups of entry trajectories belonging to commissioned composite ablation zones are used to generate ablation configurations (e.g., a set of one or more partially overlapping ablation zones lying along the groups' barycentre entry trajectory and covering the segmented target regions). In each case, differences in settings may be used to achieve composite ablations. Note that the total set of ablation configurations covering the target region may be a combination of simple and composite ablation zones. The method 90 further comprises selecting a preferred set of composite ablations (96). In an optimization embodiment for selection functionality, the huge set of generated ablation configurations are passed as an input to a heuristic iterative set covering optimization algorithm (e.g., via selection module 46, FIG. 2), which selects the preferred (e.g., minimum) set of composite (and simple) ablation zones covering the target region as much as possible, while sparing the risk organs nearby. Here, the ablation zones configuration is processed to see how well it covers the target area and spares the risk organ regions. To do this, in one embodiment, a set of ablation coverage volume-based quadratic functional objectives may be used, as disclosed in commonly-assigned international publication WO2021/032449, and incorporated by reference in its entirety.

Digressing briefly, for grid-based applications, the heuristic iterative discrete optimization method described in WO2021/032449 may be extended to support the manual positioning of the applicators using grid holes that respect a minimum distance requested by the user, or prescribed by the system itself, or other entities (e.g. applicator vendor). Hereto, firstly, the user is asked to provide a given minimum grid holes' (or probes') distance value. Secondly, when an optimal applicator configuration is selected at each inverse planning optimization iteration, all grid holes within a given distance from the currently selected hole are inactivated and made unavailable to the mathematical solver over subsequent optimization iterations. Finally, the computed optimal set of ablation probes are visualized on a suitable GUI. Accordingly, an iterative ablation optimization algorithm as extended from that disclosed in WO2021/032449 is able to enforce a threshold (e.g., minimum) distance between finally selected single applicators and ablation zones to deliver for treatment, which allows the work of the clinical expert (user) to be simplified by avoiding potential device collisions that could hamper the exact treatment execution.

The above-described idea from WO2021/032449 may be reused in method 90 for composite zones that correspond to groups of applicators as described herein. When groups of applicators are commissioned and used for treatment, the threshold distance between applicators is replaced with (replaced by) a more general threshold distance between groups of applicators, which is defined as the threshold (e.g., minimum or user or system-defined) distance between pairs of applicators belonging to the same and/or different groups (e.g., for composite ablation zones). For instance, a minimum distance d may be enforced among pairs of delivered applicators. Assuming two groups of applicators (e.g., one with a triangular placement pattern and the other with a linear placement pattern) are delivered, enforcement includes ensuring that the minimum distance d is enforced over the pairs of applicators, without any distinction if they belong to different or the same group.

During the optimization iterations of the above-described embodiment, a solver iteratively picks an optimal ablation configuration from the big set of ablation configurations created at the previous steps discussed above. Hereto, potential configurations associated to groups of applicators positioned too close to the currently selected optimal set (e.g., groups of applicators are closer than a minimum distance provided by the user) are neglected and not selected.

Alternatively, or in addition to the threshold distance between groups of applicators, another requirement may be to avoid or reduce overlaps between group patterns (e.g., having two triangular groups partially overlapping, such as shown in FIG. 4A). This event may make the treatment execution too complex, and the newly introduced threshold distance between applicators groups may not be enough to avoid such a situation. A potential solution to avoid or reduce such overlap between groups of applicators may be to develop the "point-in-polygon" check method. Given two groups of applicators, for instance, two sets of triangular insertion patterns (as shown in FIG. 3), a check may be made to assess if any of the vertices (e.g., applicator insertion points) of one triangle lies inside the other one, or not. If the check identifies an overlapping pattern, a current ablation configuration belonging to the current groups of applicators is neglected and not processed further by the optimization algorithm.

It is noted that while placing applicators inside the pattern of another composite ablation zone is not desired, re-using applicators positioned to deliver one composite ablation zone to deliver another composite ablation zone may be useful in certain clinical usage scenarios. Accordingly, some embodiments of method 90 may enable such multiplicity, re-using of virtual applicators to contribute to multiple composite ablation zones.

Though the method step 96 is described primarily above in the context of an optimization method, it should be appreciated that a preferred selection may be achieved based on a truncated optimization and/or one or more criteria as described above, where the preferred selection may be different than a fully-performed, optimization-driven result.

In some embodiments of an ablation therapy system (e.g., ablation therapy system 12, FIG. 2), interactive, manual planning of composite ablations may be supported by providing the user with the ability to either virtually position composite ablation zones and derive associated applicator positioning, or virtually position applicators and derive a composite ablation zone if possible. For instance, the user may position some groups of probes and select the composite ablation zone to deliver treatment (e.g., produced a commissioned (based on power, time) pair to use). This positioned, blended composite ablation zone may be used to initialize the iterative selection (e.g., from step 96 in FIG. 8). In some embodiments, the composite ablation zone may already cover some parts of the target region, so selection functionality focuses on selecting new probe groups to cover the remaining uncovered parts. In some embodiments, the user may commission a number of devices with different placement patterns, which may be provided in a GUI for user selection. In some embodiments, the GUI may trigger an error indication when an unfeasible pattern is selected not matching the grid geometry. In one embodiment, the user may position a composite ablation zone using a given user interface. In some embodiments, the above-described deriving of applicator positioning or composite ablation zones may be automated based on the user's initial input, as described above. Here, the corresponding group of applicators (e.g., with a triangular insertion pattern) may be positioned (by the system) and adjusted (e.g., rotated around the composite ablation zone insertion axis) by the user using suitable user interface tools (e.g., a virtual or physical scroll wheel, etc.). For example, the user may change the ablation shape (e.g., via adjustment in power/duration settings) to deliver over a positioned group, and/or the user may rotate, when possible, the group placement around the composite ablation zone axis.

In some embodiments, after the user positions a composite ablation zone using a given user interface, a dedicated selection process (e.g., an automatic optimization loop) may be executed to search a preferred (e.g., an optimal) rotation angle to use for the positioning of the corresponding group of applicators.

In embodiments where the user is enabled to virtually position individual applicators, an associated composite ablation zone may be proposed by the system. The user may have control of selecting different composite ablation zones that match the current positioning of virtual applicators (e.g., change the ablation zone shape via a change in settings).

In some embodiments, the user may be guided in the virtual positioning of subsequent applicators based on the potential composite ablation zones to be used using the same or different patterns (or in the case of grid-less treatment, over the same or different virtual boxes). For instance, once a first applicator is virtually positioned, only a few virtual locations may be used to achieve the commissioned composite ablation zones. These few virtual locations may be visualized using overlay graphics.

Note that one or more of these interactive planning methods may be used to plan complete ablation procedures (i.e., from scratch), as well as to correct/adjust ablation plans calculated by the automated planning component.

Method 90 further comprises guiding (e.g., via guidance module 48) placement of applicators based on selected composite ablation zones (98). Applicator placement may be guided by a display of overlay graphics on top of per-operative medical imaging, including, but not limited to, ultrasound imaging (e.g., 2D/3D in real-time), fluoroscopy (real-time), magnetic resonance (e.g., MR, in 2D/3D real-time or static), computed tomography (e.g., CT (3D static)), or cone beam CT (e.g., CBCT (3D static)). In addition, the per-operative imaging may be complemented with fusion of pre-operative imaging to visualize otherwise invisible target regions. Such pre-operative imaging may include, but is not limited to, MR, CT, single photon emission CT (SPECT), or positron emission tomography (PET). Applicator placement may not be according to plan in some instances, and so the ablation therapy system includes a specified behavior for dealing with placement inaccuracy. In one embodiment, in case placement deviates from the plan, the system may raise an alarm based on a fixed threshold for placement error, raise an alarm based on a configurable threshold for placement error, or have no alarm. In addition, the system may still visualize an anticipated ablation zone relative to the average tip location and orientation, an anticipated ablation zone according the initial plan (i.e., remains unchanged), or may not visualize an anticipated ablation zone.

Guidance may further be complemented with adaptive plan improvements, effectively re-computing the selected plan for the remainder of the procedure given already placed or performed ablations. In case only some of the applicators associated with a composite ablation zone are placed, and others are planned, adaptive planning may re-select the composite ablation using the (average) orientation and depth of the placed applicator(s), or keep the composite ablation as is, and only select positioning of applicators associated with other ablation zones.

Finally, the method 90 comprises performing ablation therapy based on the selected composite ablation zones (100), which is existing in the field and hence further discussion of the same is omitted here to avoid obfuscating more relevant features of the ablation therapy method 90.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope.

**Claims**

1. A system (12), comprising:

   a memory (20) comprising instructions (32); and
   one or more processors (16) configured by the instructions to:
   commission plural ablation zones (82, 84) for a target region (58A), wherein at least one of the plural ablation zones comprises a composite ablation zone based on a blending of a group of simulated ablation zones using implicit functions.

2. The system of the preceding claim, wherein the one or more processors are further configured by the instructions to commission the composite ablation zone by computing a group of entry trajectories (50) corresponding to applicators for the group of simulated ablation zones within a bounding box (54).

3. The system of any one of the preceding claims, wherein the bounding box comprises:

   a virtual plane and wherein the one or more processors are further configured by the instructions to shift, rotate, or shift and rotate the group of entry trajectories within the bounding box; or
   a grid template (64) and the one or more processors are further configured by the instructions to compute possible insertion patterns for the group of entry trajectories based on shifts in an x-direction, y-direction, or both x and y directions.

4. The system of any one of the preceding claims, wherein the one or more processors is further configured by the instructions to adjust the blending according to one or more blending parameters that are set directly based on user input or according to one or more blending parameters that are set indirectly based on selection of the one or more blending parameters that mimics a composite ablation shape prescribed by a mesh.

5. The system of any one of the preceding claims, wherein the composite ablation zone comprises a non-ellipsoidal shape (10A).

6. The system of any one of the preceding claims, further comprising a user interface (24), wherein the one or more processors are further configured by the instructions to provide a graphical user interface (34) on the user interface to enable a user to instruct commissioning of the plural ablation zones.

7. The system of any one of the preceding claims, wherein the one or more processors are further configured by the instructions to select, from among the plural commissioned ablation zones, one or more of the commissioned ablation zones for use in ablation therapy treatment while removing one or more of the commissioned ablation zones from consideration in the treatment.

8. The system of any one of the preceding claims, wherein the one or more processors are further configured by the instructions to select at least the composite ablation zone based on an optimization function.

9. The system of any one of the preceding claims, wherein the one or more processors are further configured by the instructions to perform the selection by enforcing one or more requirements.

10. The system of any one of the preceding claims, wherein enforcing the one or more requirements comprises establishing a threshold distance between pairs of applicators corresponding to the plural commissioned ablation zones or reducing overlap between different groups of applicators.

11. The system of any one of the preceding claims, wherein the one or more processors are further configured by the instructions to enable guidance of the plural applicators corresponding to the composite ablation zone in the target region via providing a graphical user interface with overlay graphics on top of per-operative medical imaging.

12. The system of any one of the preceding claims, wherein the one or more processors are further configured by the instructions to provide an alarm when guided placement of the plural applicators deviates from plan, wherein the alarm comprises one of a fixed threshold for placement error or a configurable threshold for the placement error.

13. The system of any one of the preceding claims, wherein the one or more processors are further configured by the instructions to re-select one or more composite ablation zones after one or more applicators are physically placed in the target region.

14. A method (90) for implementing the functions of any one of the preceding claims.

15. A non-transitory, computer readable storage medium (20) comprising instructions that when executed by the one or more processors, causes the one or more processors to implemented the functions of any one of the preceding claims.

10A →  FIG. 1A

10B →  FIG. 1B

10C →  FIG. 1C

EP 4 437 991 A1

FIG. 2

**FIG. 3**

**FIG. 4A**

**FIG. 4B**

FIG. 5B

FIG. 5A

FIG. 6

**FIG. 7A**

**FIG. 7B**

**FIG. 7C**

EP 4 437 991 A1

90

START

92 COMPUTE GROUPS OF ENTRY TRAJECTORIES

94 GENERATE ABLATION CONFIGURATIONS USING THE COMPUTED GROUPS

96 SELECT PREFERRED SET OF COMPOSITE ABLATION ZONES

98 GUIDE PLACEMENT OF APPLICATORS BASED ON SELECTED COMPOSITE ABLATION ZONES

100 PERFORM THERMAL ABLATION THERAPY

END

**FIG. 8**

Europäisches
Patentamt

European
Patent Office

Office européen
des brevets

# EUROPEAN SEARCH REPORT

Application Number

EP 23 16 6058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2022/265359 A1 (ISOLA ALFONSO AGATINO [NL] ET AL) 25 August 2022 (2022-08-25)<br>* paragraph [0092] – paragraph [0093]; figure 1 *<br>* paragraph [0117] – paragraph [0122]; figure 6 *<br>* paragraph [0197] – paragraph [0201]; figures 23-24 *<br>* paragraph [0129]; figure 8 *<br>* paragraph [0138] *<br>* paragraph [0170] *<br>----- | 1-11, 13-15<br>12 | INV.<br>A61B34/10<br>A61B18/14<br><br>ADD.<br>A61B34/20<br>A61B90/00 |
| X<br><br>A | US 2021/007805 A1 (LIU XIN [US] ET AL) 14 January 2021 (2021-01-14)<br><br>* paragraph [0052] – paragraph [0056]; figures 5-6 *<br>* paragraph [0041] *<br>----- | 1,2, 4-11,14, 15<br>3,12,13 | |
| X<br><br>A | JP 2016 052541 A (SMITH & NEPHEW INC) 14 April 2016 (2016-04-14)<br>* paragraph [0044] *<br>* paragraph [0047] *<br>----- | 1,12,14, 15<br><br>2-11,13 | TECHNICAL FIELDS SEARCHED (IPC)<br><br>A61B |

The present search report has been drawn up for all claims

| Place of search | Date of completion of the search | Examiner |
|---|---|---|
| Munich | 11 August 2023 | Geiss, Oana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 16 6058

This annex lists the patent family members relating to the patent documents cited in the above-mentioned European search report.
The members are as contained in the European Patent Office EDP file on
The European Patent Office is in no way liable for these particulars which are merely given for the purpose of information.

11-08-2023

| Patent document cited in search report | | Publication date | Patent family member(s) | | | Publication date |
|---|---|---|---|---|---|---|
| US 2022265359 | A1 | 25-08-2022 | CN | 114302687 | A | 08-04-2022 |
| | | | EP | 3777748 | A1 | 17-02-2021 |
| | | | EP | 4013332 | A1 | 22-06-2022 |
| | | | US | 2022265359 | A1 | 25-08-2022 |
| | | | WO | 2021032449 | A1 | 25-02-2021 |
| US 2021007805 | A1 | 14-01-2021 | CN | 103717167 | A | 09-04-2014 |
| | | | EP | 2736436 | A1 | 04-06-2014 |
| | | | JP | 6129833 | B2 | 17-05-2017 |
| | | | JP | 2015500664 | A | 08-01-2015 |
| | | | US | 2014201669 | A1 | 17-07-2014 |
| | | | US | 2021007805 | A1 | 14-01-2021 |
| | | | WO | 2013014648 | A1 | 31-01-2013 |
| JP 2016052541 | A | 14-04-2016 | AU | 2011239570 | A1 | 01-11-2012 |
| | | | CN | 102933163 | A | 13-02-2013 |
| | | | EP | 2558011 | A2 | 20-02-2013 |
| | | | JP | 2013523415 | A | 17-06-2013 |
| | | | JP | 2016052541 | A | 14-04-2016 |
| | | | US | 2011257653 | A1 | 20-10-2011 |
| | | | US | 2018153624 | A1 | 07-06-2018 |
| | | | US | 2022151712 | A1 | 19-05-2022 |
| | | | US | 2023157764 | A1 | 25-05-2023 |
| | | | WO | 2011130567 | A2 | 20-10-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021032449 A